# EUROPEAN PATENT APPLICATION

(11) **EP 4 541 334 A1**
(43) Date of publication of application: **23.04.2025**
(21) Application number: 24207283.3
(22) Date of filing: 17.10.2024
(51) Int. Cl.: A61F 13/551, B65D 85/07

(54) **ABSORBENT ARTICLE PACK WITH HOLDING BAND & METHOD FOR PACKING ABSORBENT ARTICLES**

(30) Priority: 18.10.2023 NL 2036066
(71) Applicant: Drylock Technologies NV, 9240 Zele (BE)
(72) Inventor: Speeleveld, Xavier, 9240 Zele (BE); Derycke, Tom, 9240 Zele (BE)
(74) Representative: Arnold & Siedsma

(57) **Abstract**

An absorbent article pack with one or more absorbent articles held by a holding band, the holding band is arranged around the one or more absorbent articles to hold them together. The disclosure further relates to a method for packing one absorbent article or a plurality of absorbent articles, the method comprising arranging at least one holding band around the one or more absorbent articles such that the absorbent article is, or the more absorbent articles are held together by the at least one holding band.

## Description

### TECHNICAL FIELD

The present invention relates to the technical field an absorbent article pack and a method of packing one or more absorbent articles. The absorbent article can be for example a personal hygiene product, a diaper, a panty liner or the like.

### BACKGROUND

Absorbent articles, such as diapers or other personal hygiene products, can be disseminated through various channels. Traditionally, diapers are bundled within plastic encasements for transportation from the factory to the customer.

Nevertheless, the conventional practices of packaging and transporting these absorbent articles have raised environmental concerns due to the generation of waste materials and the use of plastic materials. Consequently, there is a compelling necessity to optimize and revolutionize how absorbent articles are transported and/or offered for sale, with a focus on sustainability.

### SUMMARY

It is an object of embodiments of the present disclosure to improve how absorbent articles are transported and/or offered for sale, in particular with a focus on sustainability, preferably while at the same time having a focus on delivering the absorbent articles to an end consumer without defects.

Thereto, a first aspect provides an absorbent article pack comprising one or more absorbent articles and at least one holding band. The band is arranged around one or more absorbent articles such that the one or more absorbent articles are held together by the at least one holding band.

By utilizing the holding band to hold the article(s) together, the sustainability of transporting the article(s) can be improved. Namely, the need for traditional plastic bags, which typically completely wrap a number of absorbent articles, to transport and/or offer for sale the article(s) can be omitted (using no bag) or reduced (using less raw material for the bag). As a result, the total mass of packaging materials can be reduced which allows for an improvement of sustainability as a result of less waste.

Furthermore, the holding band increases the reliability that the articles are kept in a desired condition during transport and/or offering for sale, in particular in case the articles are folded and/or compressed. For example, in case a packaging bag is ruptured, the holding band can still keep the articles together in a desired fashion. This way, packaging and/or transport defects can be reduced.

Preferably, the at least one holding band is arranged in direct contact with the one or more absorbent articles. The expression "in direct contact" refers to not having any sheet or bag in between the holding band and the one or more absorbent articles, in particular in case the one or more absorbent articles are diapers, preferably diapers in folded condition. By not having sheet or bag, the amount of packaging materials can be decreased which allows for a more sustainable outcome.

In some embodiments, an encasement may be desired for hygiene purposes, e.g. in case the one or more absorbent articles are panty liners or menstrual pads. In such embodiments it is preferred that both the absorbent article(s) and the encasement are in folded condition. More in particular, according to an embodiment, a plurality of absorbent articles are arranged in a stack with each absorbent article individually encased by an encasement and subsequently folded such that the folded encased absorbent articles are held by the one or more holding bands.

An encasement may be arranged inside or outside the holding band. Or with other words, the holding band may be arranged within the encasement or around the encasement. By having the holding band within the encasement, the band may enjoy an additional protection by the encasement which reduces change of packing defect during transport. By having the band present, the need for further encasements may also be reduced which benefits sustainability.

Preferably, the band may have a width within the range of 0,2 cm to 10 cm, preferably within the range of 0,5 cm and 8 cm, more preferably within 0,5 cm and 6 cm. The width may vary depending on the number and type of articles to be held by the band. By having the width as described, the article(s) can be held in a reliable manner with an efficient use of raw material for the band.

Additionally, it was surprisingly found that product recognition could be improved as a result of the articles being more visible to customers. In particular when the holding band is applied around the one or more absorbent articles while allowing a direct view of the one or more absorbent articles within the pack, e.g. in an embodiment without any packaging(s) and/or sub-packaging, like a plastic bag.

Preferably, the one or more absorbent articles comprise a plurality of absorbent articles which are arranged in a stack, and wherein the at least one holding band is arranged around the stack and preferably wherein the holding band is in direct contact with the one or more absorbent articles. In this manner, the transport material per unit of article can be reduced, which allows for an improvement in sustainability and a reduction in cost.

Preferably, the least one holding band is arranged such that the one or more absorbent articles are held together in at least a partially compressed state. By doing so, more articles can be transported per action of transport which results in an improvement in sustainability and a reduction in cost. Namely, compressing absorbent articles can reduce the amount of space they occupy, making it easier to store them in limited or confined spaces. This is particularly useful to maximize storage efficiency.

In a general sense, the term "compressed" refers to the action or process of reducing the volume or size of an object or material by applying force or pressure to it. When the article(s) is/are compressed, it is typically made more compact or dense compared to its natural or initial state. The article(s) can be compressed by tightening the holding band and/or by pre-compressing the articles before the holding band is applied. Surprisingly, by having the article(s) in compressed state, they remain more securely within the holding band as they exert an outward pressure on the holding band which causes the article(s) to stay more reliably packed.

Preferably, the one or more absorbent articles are folded and are kept in folded condition by aid of the at least one holding band. In this folded manner, the articles can be transported more efficiently. Additionally, the folded condition is kept more secure over time by aid of the holding band. With other words, accidental loss of the folded condition is avoided by the holding band. This is beneficial since the folded condition can help protect article(s) from damage during transportation or any other impact. Also, when the articles are neatly folded and stacked, there's less chance of them shifting during transit.

The absorbent articles may be bulky, occupying a significant amount of space during storage and transportation. It was found that the absorbent articles can transported in a more efficient manner when folded. The articles can be folded in various ways, e.g. via one or two folding lines. Diapers in particular are preferably folded via at least one folding line resulting in a u-shape or v-shape when seen through a cross section of the folded articles. Panty liners and/or menstrual pads and/or sanitary napkins can be folded in various manners as well, preferably folded via at least two folding lines. Preferably, each liner or pad or napkin is individually encased and subsequently arranged in a stack. The stack of individually encased articles is then held by the one or more holding bands.

Preferably, each of the one or more absorbent articles in folded condition comprise at least two folding lines. In this manner, a more portable way is ensured. According to an embodiment, the one or more absorbent articles each have a body with a first end portion and a section end portion, wherein for each of the articles, the body is folded such that the first end portion overlaps with the second end portion. By overlapping the end portions, the portability of the respective article is improved, which is desired in particular when there is intention to discreetly carry the article, e.g. in a handbag or a pocket. The one or more absorbent articles can be chosen from sanitary napkins, in such a case, it preferred that the sanitary napkins are each individually packed via a folded encasement that is folded such that folding lines of the encasement correspond to folding lines of the encased body. Preferably at least one, more preferably at least two folding lines are present which extend substantially perpendicular to the longitudinal axis of the body of the respective sanitary napkin. The folding of both the encasement and the sanitary napkin can be achieved by firstly encasing the sanitary napkin and subsequently folding the encased napkins so that the folding lines overlap.

According to a preferred embodiment, the absorbent articles are individually encased and subsequently folded before being arranged in a stack. In this manner, the article and encasement are folded together. This embodiment is found to be highly beneficial for absorbent articles being panty liners or menstrual pads.

A particular aspect provides for an absorbent article pack comprising an absorbent article or a plurality of absorbent articles, wherein the article or each individual article within said plurality of articles is encased by a corresponding individual encasement; and wherein the article or each article and its corresponding individual encasement are folded together thereby forming individually folded encased articles, wherein said individually folded encased article(s) are held by a holding band. Preferably, the folded encased articles are arranged in a stack of individually encased folded articles and whereby the holding band is arranged around the stack to hold the stack of encased folded articles. By having the article or each article within the plurality article in a folded condition, a more portable arrangement of each article can be achieved while the articles can be distributed in a more sustainable manner. This aspect is found to be highly beneficial for absorbent articles being panty liners or menstrual pads, also described herein as sanitary napkins.

The holding band can be applied around multiple articles and can be resealable. This way, an article can be taken away and the holding band can be used again to hold the remaining articles.

In an embodiment, the pack further has an encasement encasing the one or more absorbent articles. The encasement can be used to improve hygiene and/or to protect the articles from impacting factors during transport. The particular combination of having the holding band allows for a reduction in thickness of the encasement. This can result in less use of transport materials which improves overall sustainability.

Each article can be packed within the encasement individually or together in a group. The holding band can be placed around the encasement encasing the article(s) or the holding band can be placed directly around the article(s) having the encasement encasing the article(s) held together by the band. Notably, a combination of both ways is also possible.

In another embodiment, the absorbent article pack is free of any bags and/or sub-encasements. In this manner, overall use of transport material can be reduced which benefits sustainability.

In an embodiment, the at least one holding band holding together the one or more absorbent articles is arranged within the encasement. This way, the holding band is protected by the encasement. In addition thereto, or alternatively, an encasement encasing one or more absorbent articles is arranged within the at least one holding band. With other words, the holding hand is then arranged outside said encasement. Generally, by having both an encasement and the holding band present, the material needed of each component, in particular the material of the encasement needed can be reduced which benefits sustainability. Namely at least a part of the strength needed for a reliable holding of the article(s) is provided by the band as a result, the thickness and/or the strength of the encasement can be reduced.

If an encasement is present, then this encasement preferably has a thickness (t) smaller than 500 micron. Conventional encasements, such as a plastic bag, typically need a thicker thickness to keep the article(s) in a preferred state or condition, which can be a compressed state or a folded condition. Surprisingly, a reliable securement of such state or condition can now be provided even with a thinner encasement due to the holding band. More preferably, the thickness of the encasement is smaller than 50 micron, preferably between 5-50 micron, more preferably 6 - 40 micron, even more preferably between 7-30 micron, most preferably between 8-20 micron, such as 10 micron. The encasement may be partially or fully transparent such that to the inside content is visible. In this manner, product recognition can be improved.

Generally, it is preferable that the at least one holding band comprises a dedicated surface provided with information related to the one or more absorbent articles. This way, a customer can be better informed on the absorbent articles.

The holding band can be a any suitable means to hold the one or more absorbent articles, examples thereof are a rope or a strap for example manufactured with a plastic and/or a paper material.

Preferably, at least one holding band comprises a band made of a paper material. In this manner, the resulting pack is considered more sustainable allowing a reduced environmental impact of the article(s) being transported. Preferably, the paper material is unbleached and/or uncoated paper. As such, sustainability can be enhanced due to a reduced chemical use and/or due to energy savings during production. Additionally, customer appeal is increased since many environmentally conscious consumers prefer packaging that visibly demonstrates sustainability efforts, making unbleached and uncoated paper an attractive choice.

Preferably, the paper material of the holding band has a grammage of between 50 g/m2 and 150 g/m², preferably between 60 g/m² and 140 g/m², more preferably between 70 g/m² and 140 g/m², most preferably between 80 g/m² and 120 g/m². As such, sustainability can be enhanced as a result of an effective use of raw materials to develop the holding band. It was found that higher grammages may impact environmental considerations while lower grammages could impact durability considerations. Surprisingly, a durable and sustainable result can be achieved while the holding band allows for printing and branding thereon within the chosen range of grammage. On top of that, it was found that manufacturing errors with the paper material could be reduced and even avoided.

The at least one holding band comprises may be a band (200) made of plastic material. In this manner, a reliable and strong band can be applied which provides for a good holding of the articles. The at least one holding band (200) can be made from a material chosen from the group consisting of polyethylene (PE), polypropylene (PP), polyvinyl chloride (PVC), polyethylene terephthalate (PET), polyamide, ethylene vinyl alcohol (EVOH), polylactic acid (PLA) and/or manufactured form a coextruded film; preferably wherein the at least one holding band is made from polypropylene (PP).

As said, an encasement can be used to encase individual or a group of articles. In a preferred embodiment, the encasement is a shrink film as it allows for high amount of articles to be transported in a confined space. In this manner, more articles can be transported per transport action. Additionally, the film may be applied in a rapid manner.

The encasement may be made from a material chosen from the group consisting of polyethylene (PE), polyvinyl chloride (PVC), polyethylene terephthalate (PET), polylactic acid (PLA) or Polypropylene (PP); and/or is manufactured from a coextruded film, wherein preferably the encasement is made from polyethylene (PE). These materials may provide for a desired protection against environmental factors. Preferably, the encasement is made from a biodegradable material, preferably polylactic acid (PLA). In this manner, sustainability can be enhanced.

Preferably, at least one holding band and/or the encasement are made from a recycled material, preferably a post-consumer recycled (PCR) material, such as a recycled PE material or a recycled PET material. In this manner, sustainability can be improved.

Preferably, the encasement is transparent so as to allow visual observation the one or more absorbent articles within the encasement.

Preferably, the at least one holding band is provided with a resealable closure mechanism. The resealable closure mechanism of the band, preferably a paper band, provides a convenient and efficient way to re-secure the article(s) after the band has been opened.

As a result, the customers such as care givers are allowed to better organize the remaining articles after having removed one. The resealable closure mechanism can include any one of a pressure sensitive adhesive, a hook and loop system, a snap closure system, a fold-over-flap system or any combination thereof.

In an embodiment, the at least one holding band has ends that are sealed together. The band has typically two opposing ends that are sealed together. The ends can be sealed via heat sealing and/or via ultrasonic sealing.

In an embodiment, the at least one holding band is provided with a thermoplastic film or coating at least at the ends of the holding band. When heated, e.g. by using a thermal or ultrasonic heater, the thermoplastic film or coating can bond and seal the ends of the holding band together.

Multiple bands can be present for a more secure holding of the articles. In an embodiment, the at least one holding band comprises a first holding band and a second holding band which are arranged around the one or more absorbent articles such that the one or more absorbent articles are held together by the first and second holding band. In this manner, the article(s) can be held in a more secure manner.

In an embodiment, the first and second holding band are arranged at a distance from each other. Preferably, the first and second holding band are arranged substantially mutually parallel. With other words, the first holding band and second holding band are applied substantially parallel to each other. By applying the bands parallel to each other, the pack can be held together by the first and second bands in a more rapid manner as seen from a production standpoint.

In another embodiment, the second holding band crosses over the first holding band. The first and second band may or may not be attached to each other. Additional holding bands can be present for a more secure holding of the article(s), e.g. two parallel bands and one band crossing the two parallel bands.

According to an embodiment, the absorbent article pack further comprises a first dimensionally stable element, and optionally a second dimensionally stable element, positioned between the at least one holding band and the one or more absorbent articles and/or between two or more absorbent articles of the one or more absorbent articles. The dimensionally stable element may be a cardboard piece.

The stability of the pack may be increased by providing one or more dimensionally stable elements within the encasement and/or within the holding band, next to the article(s). For example, one or more dimensionally stable elements may be positioned adjacent and/or in between the absorbent articles. The dimensionally stable elements may be provided with data such as product information, brand information and/or logo, which can improve customer recognition of the absorbent articles. The provision of one or more dimensionally stable elements might also improve the appearance of the pack. Preferably, the pack comprises a first dimensionally stable element, such as a cardboard piece or other suitable elements causing dimensional stability when the articles are held together by the holding band. A second dimensionally stable element may be provided, the first and second stable elements can be placed at the sides of the pack and can be held together via one or more holding bands. Including stable elements may also improve the stacking capability of the pack. In particular in case that one or more dimensionally stable elements are placed within the pack adjacent to an article and/or in between articles.

The absorbent article(s) can be chosen from an article that comprises or the articles within the plurality of absorbent articles each comprise a liquid pervious topsheet, a liquid impervious backsheet, absorbent material positioned between the liquid pervious topsheet and the liquid impervious backsheet, and a bottom layer between the absorbent material and the liquid impervious backsheet, wherein the absorbent material comprises superabsorbent particles and/or cellulosic fluff pulp and wherein the absorbent material is arranged such that one or more channels are formed, wherein less absorbent material per surface area is present in the one or more channels compared to an area around the one or more channels, wherein preferably substantially no absorbent material is present in the one or more channels, wherein the bottom layer is in contact with the absorbent material and has an average density as measured in a decompressed state between 20 and 400 kg/m3, preferably between 20 and 300 kg/m3, more preferably between 20 and 250 kg/m3, and even more preferably between 20 and 200 kg/m3, e.g. between 20 and 120 kg/m3. The chosen average density results in a fluffy feel which improves the comfort of the user.

A further aspect provides for a container, such as a box or a bag, containing one or more absorbent article packs as described herein. By having such container, the pack can be transported with a more sustainable and possibly more cost effective manner. Namely, then need and/or material for plastic bags can be reduced or omitted as a result of the holding band.

Preferably, a plurality of the absorbent article packs are arranged directly adjacent to each other within the container and are preferably stacked one above the other and/or are arranged next to each other. Such arrangement may benefit a more sustainable way of transport per article unit.

A further aspect provides for a method for packing one absorbent article or a plurality of absorbent articles. The method comprises arranging at least one holding band around the one or more absorbent articles such that the absorbent article is or the more absorbent articles are held together by the at least one holding band.

Understandably, the technical benefits in connection to the holding band or a corresponding element are equally applicable in this aspect providing the described method. By utilizing the holding band to hold the article(s) together, the sustainability of transporting the article(s) can be improved. On top of day, many consumers prioritize eco-friendly products. Using a more sustainable way to distribute the articles can help to improve environmental aspects which can help to attract and retain customers who are environmentally conscious.

Preferably, the arranging comprises, arranging the at least one holding band in an open state around the one or more absorbent articles and sealing together a first and second end of the at least one holding band to achieve a closed state of the at least one holding band. In this manner, the holding band can be applied rapidly and securely around the absorbent article(s). The sealing together is preferably done by heat-sealing or ultrasonic sealing. The holding band may comprise a thermoplastic material to facilitate such sealing.

Preferably, the one or more absorbent articles are folded and are subsequently encased and/or held by the at least one holding band. The articles can thus be folded before being encased. It is preferred that the articles are folded to achieve a more compact result.

According to an embodiment, the one or more absorbent articles are encased and subsequently folded, preferably folded via at least two folding lines located such that end portions of the encased absorbent article overlap. By folding the encased articles, a more compact result is achieved which helps to discreetly carry the articles. Such folding of encased articles is beneficial, in particular for sanitary napkin.

The method preferably further comprises: - compressing the one or more absorbent articles. By including a compressing action, more articles can be transported per unit of space. Namely, the compressing action induces a reducing of volume or size of one or more absorbent articles. Compressing can be done by applying pressure and/or by using machinery to compact the articles. In addition or as an alternative, the article(s) can be compressed by tightening the holding band. By compressing the absorbent articles, they take up less space relative to their original, uncompressed state. This means that more articles can fit into the same transportation container or space. Using space more efficiently can contribute to resource conservation. It can further lead to reduced use of packaging materials and lower shipping costs.

The one or more absorbent articles can be compressed before being surrounded by the at least one holding band and/or the one or more absorbent articles can be compressed by being surrounded by the at least one holding band and by subsequently tightening the at least one holding band.

In the case that the one or more absorbent articles are compressed before being surrounded by the at least one holding band, then the holding band may help to keep the compressed state of the articles over a prolonged period and/or the holding band may help to absorb expanding forces of the article. By doing so, the holding band allows that encasements can be made thinner which reduced material use. The article(s) can be compressed before being surrounded by the at least one holding band by compressing them with a compressor or by including the article(s) in a vacuum sealable encasement and vacuum sealing the article(s). This can lead to reduced transportation costs per unit as more units can be transported per transport vessel.

The holding band itself may also be used to compress the article(s), in this case after the holding band is placed around the articles, there is a further action to tighten it via the holding band. By tightening the holding band, the articles can be compressed.

In an embodiment, the absorbent article or the plurality of absorbent articles in an uncompressed state take up a total volume V1, wherein the one or more absorbent articles are compressed such that the total volume V1 of the plurality of absorbent articles is reduced by a factor of 1.05, preferably by a factor of 1.1, more preferably by a factor of 1.2. By doing so, one significantly reduces the space they occupy, allowing for more efficient storage, transportation, and utilization of available resources.

In a particular embodiment, the article(s) are compressed by vacuum sealing. The vacuum sealing can be performed so as to reduce the total volume V1 of the plurality of absorbent articles by a factor of 1.4. By doing so, one significantly reduces the space they occupy, allowing for more efficient storage, transportation, and utilization of available resources.

In an embodiment, the method further comprises folding the one or more absorbent articles before arranging the holding band, and subsequently arranging the holding band such that the one or more absorbent articles are held in a folded condition by the holding band. The folded condition can help protect article(s) from damage during transportation or any other impact. The holding band can facilitate keeping this folded condition.

In an embodiment, the method further comprises providing information on the holding band, preferably by printing information on the holding band. In this manner, a consumer can be better informed.

In an embodiment, the method further comprises encasing the one or more absorbent articles in an encasement, preferably an air-impermeable encasement. An air-impermeable encasement improves vacuum sealing.

The encasement can be a shrink film and wherein the method comprises, according to an embodiment, shrinking the encasement after the one or more absorbent articles are encased within the encasement.

The holding band can be arranged around the encasement after the one or more absorbent articles are encased therein; or the holding band can be is arranged around the one or more absorbent articles before the one or more absorbent articles are encased such that the holding band and the one or more absorbent articles are encased within the encasement.

In an embodiment, the method further comprises including a first dimensionally stable element, and optionally a second dimensionally stable element, within the encasement. As set out earlier herein, including dimensions table elements may have benefits.

### BRIEF DESCRIPTION OF FIGURES

Aspects of the invention will now be described with more details with respect to the drawings illustrating some preferred embodiments.
Figure 1 shows an absorbent article held by a holding band.
Figure 2 shows a group of absorbent articles held by a holding band.
Figures 3A-3B shows an absorbent article held by a holding band encased together by an encasement. Figure 3B illustrates that the encasement can be vacuum sealed.
Figure 4 illustrates that both the absorbent article and the encasement are held together by the holding band.
Figures 5A-5B illustrates a group of articles encased by an encasement wherein a band is arranged around respectively inside and outside the encasement.
Figure 6A-6B illustrates a band having a closure mechanism, whereby figure 6A shows a frond view and figure 6B shows a top view.
Figure 7A-7B illustrates a band of which the ends are attached to each other, whereby figure 7A shows a front view and figure 7B shows a top view.
Figures 8A-8B illustrate a technique of arranging a band around an absorbent article pack.
Figure 9 illustrates a perspective view of a group of absorbent articles held by a holding band.
Figures 10A-10D illustrate a group of absorbent articles held by one or more holding bands, whereby figure 10A illustrates a front view and figures 10B-10D illustrate perspective views.
Figures 11A-11D illustrate several examples of possible holding band and encasement arrangements.
Figure 12 illustrates a front view of a pack held by two holding bands.
Figures 13A-13B illustrate a pack of panty liners held by a holding band.
Figure 14 illustrates a container containing several absorbent article packs each individually held by a holding band.
Figures 15A-15B illustrates an absorbent article pack held by a holding band, the pack including a dimensionally stable element.
Figures 16A-16D illustrate an encased sanitary napkin, in unfolded and folded conditions, and of which figures 16C and 16D illustrate the folded sanitary napkin(s) being held by a holding band.
Figures 17A-17D illustrate one or more articles folded via two folding lines and held by a holding band.

The skilled person will appreciate on the basis of the description that the invention can be embodied in different ways and on the basis of different principles. The invention is therefore not limited to the described embodiments. It is further to be understood that elements and features bearing the same reference numerals are intended to possess corresponding features and deliver comparable technical advantages.

### DETAILED DESCRIPTION

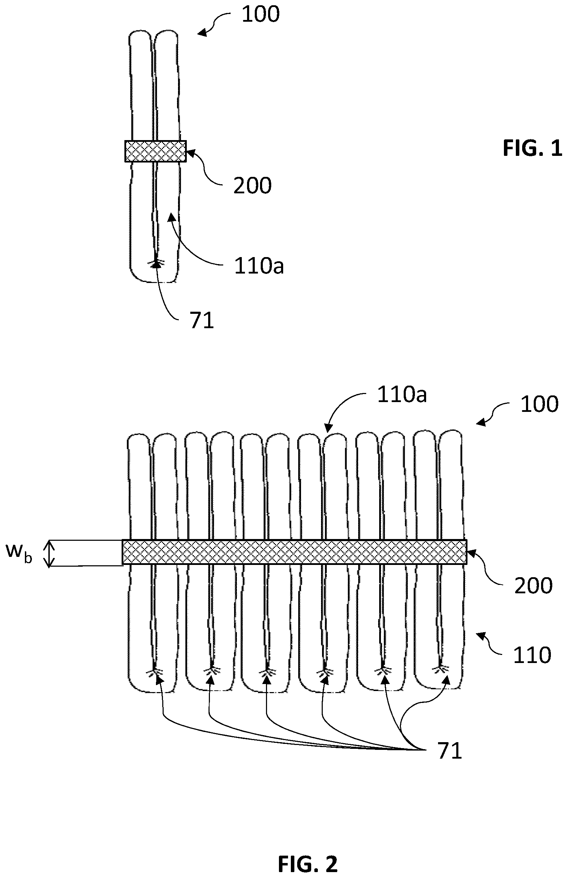

**Figure 1** shows an absorbent article pack 100 with an absorbent article 110a held by a holding band 200. The absorbent article can be any article designed to absorb and manage bodily fluids, including diapers for infants, adult incontinence products, menstrual hygiene items, wound dressings, and more. Figure 1 illustrates an example wherein the article 110a is a diaper. Generally, the holding band 200 may be a plastic or a paper band or strap or a combination thereof. Preferably, the band 200 is a paper band made of a paper material that is unbleached and/or uncoated. Also, in case of using a paper material for the holding band 200, the material preferably a grammage of between 50 g/m2 and 150 g/m2, preferably between 60 g/m2 and 140 g/m2, more preferably between 70 g/m2 and 140 g/m2, most preferably between 80 g/m2 and 120 g/m2. Preferably, to allow efficient sealing of the paper holding band, a film or coating of thermoplastic polymer is provided at least at the to be sealed parts of the holding band. A coating of polypropylene has proven to be efficient in this regard. In certain embodiments, the band 200 may be made of plastic, in particular a plastic material chosen from the group consisting of polyethylene (PE), polypropylene (PP), polyvinyl chloride (PVC), polyethylene terephthalate (PET), polyamide, ethylene vinyl alcohol (EVOH), polylactic acid (PLA) and/or manufactured form a coextruded film; preferably wherein the at least one holding band is made from polypropylene (PP). Figure 1 also indicates the absorbent article 110a being in a folded condition. The holding band 200 is placed directly around the absorbent article 110a, meaning that the band is in direct contact with the article and no encasements are placed in between. In such embodiment, the folded condition can be kept over a prolonged period with less package material by aid of the holding band.

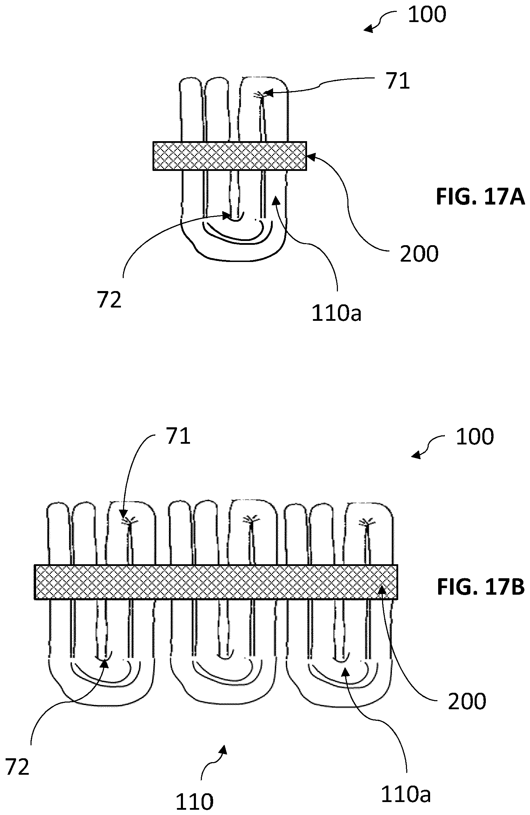

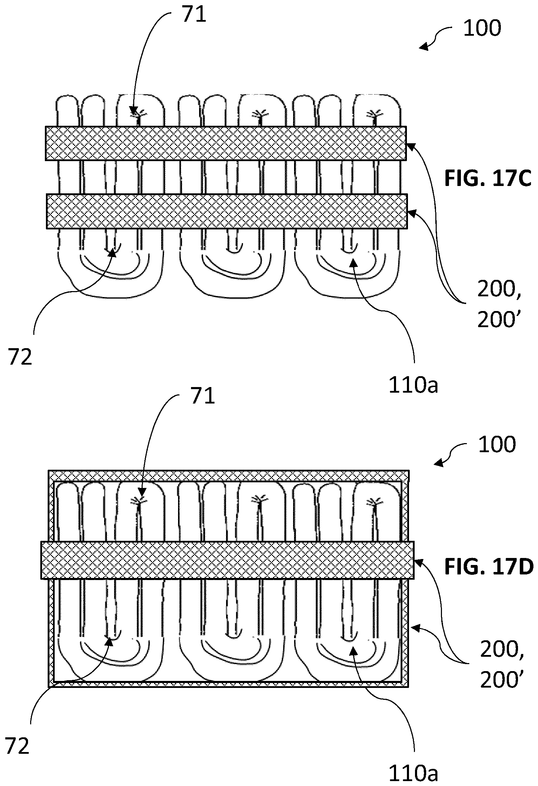

**Figure 2** shows a pack 100 with a group of absorbent articles 110 held by a holding band 200. As shown, the absorbent articles are stacked one next to each other, as such they are arranged in a side-by-side manner, with each article 110a placed alongside or adjacent to the others, creating a row or a stack of articles. The group of articles 110 are held together by holding band 200. Figure 2 further illustrates that each individual article 110a is in a folded condition and said folded condition is kept by aid of the holding band 200. The articles in figure 2 are folded via folding line 71. The band 200 can be arranged around the stack of articles in several ways with respect to the folding lines 71. As shown in figure 2, the band 200 extends in a plane that will never intersect with the folding lines or an imaginary extension thereof, shortly described the band 200 extends substantially parallel to the folding lines. In other embodiments, the band 200 may extend perpendicular to the folding lines, or with other words, the band 200 extends in a plane that intersects with the folding lines 71. A combination of two or more bands may be desired, for example for increased holding capacity and/or to firmly hold the articles (for example as shown in figures 17A-17C).

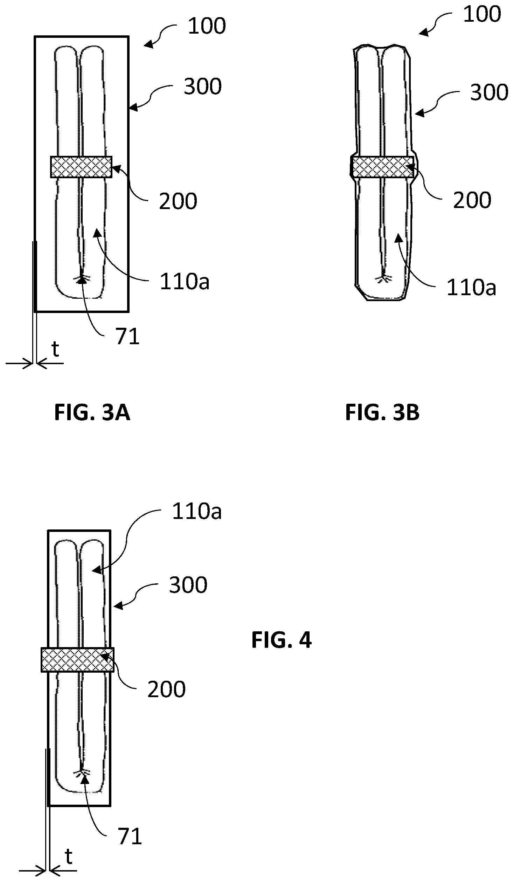

**Figure 3A** shows an absorbent article 110a held by a holding band 200 encased by an encasement 300. The holding band 200 provides for a part of the strength needed to hold the absorbent article 100a, in particular in case the article is in a compressed and/or folded condition. The encasement 300 provided additional protection against contamination of the absorbent articles. Namely, compressed and/or folded articles may exert an outward pressure. In conventional ways, an encasement with a sufficiently large thickness would be needed to address said outward pressure. By having the holding band 200 holding the article 100a, the thickness t of the encasement 300 can be reduced. This way, use of raw materials can be reduced which subsequently may improve sustainability as a result of less waste and/or lighter weight.

In figure 3A, the holding band 200 is placed within the encasement 300, with other words the band 200 is inside the encasement. In figure 4, the holding band is placed outside the encasement 300.

As for suitable materials for the encasement 300, it is preferred that a material is used chosen from the group consisting of polyethylene (PE), polyvinyl chloride (PVC), polyethylene terephthalate (PET), polylactic acid (PLA) or Polypropylene (PP).

The encasement 300 may also be manufactured from a coextruded film. Preferably, the encasement 300 is made from polyethylene (PE). For a more sustainable effect, the encasement 300 can be made from a biodegradable material, preferably polylactic acid (PLA). In this manner, sustainability can be enhanced.

Preferably, at least one holding band 200 and/or the encasement 300 are made from a recycled material, preferably a post-consumer recycled (PCR) material, such as a recycled PE material or a recycled PET material. In this manner, sustainability can be improved.

To improve customer recognition, the (outer) encasement 300 (or any sub-encasement 300a as explained further) may be configured to be transparent so as to allow visual observation the one or more absorbent articles within the encasement.

To improve transport and protection against contamination, the (outer) encasement 300 (or any sub-encasement 300a as explained further) can be a shrink film that has been applied around the articles to allow more absorbent articles 110 to be transported per unit of volume. The shrink film 300 conforms tightly to the shape of the absorbent articles creating a snug fit and may even further compress the articles.

**Figure 4** illustrates that both the absorbent article 100a and the encasement 300 are held together by the holding band 200. The band 200 is arranged around the article 100a and the encasement 200. Notably, the band 200 is outside the encasement 300. Also this embodiment can be vacuum sealed (not shown).

Preferably, the thickness t of the encasement 300 is smaller than 50 micron, preferably between 5 - 50 micron, more preferably 6 - 40 micron, even more preferably between 7-30 micron, most preferably between 8-20 micron, such as 10 micron. By having a smaller thickness t for the encasement 300, less packaging material can be used for the encasement which may benefit sustainability.

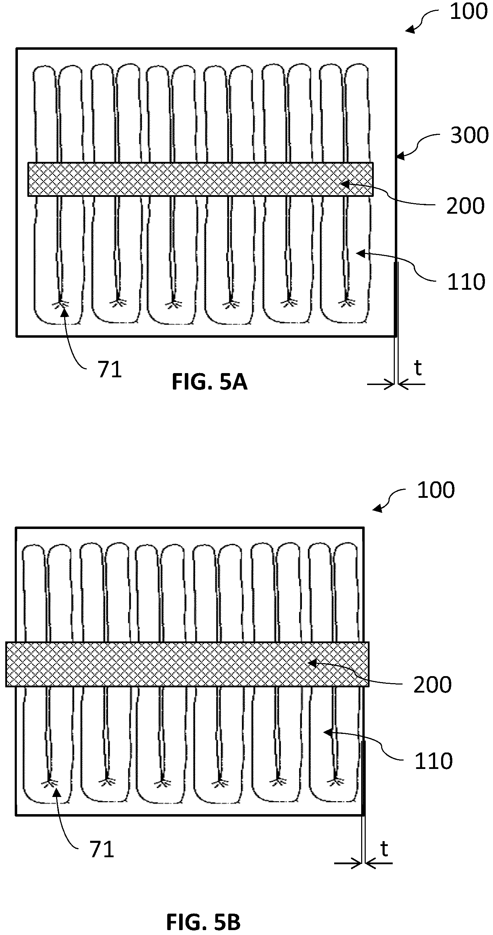

**Figures 5A** shows a pack 100 comprising multiple absorbent articles 110 that are in a folded condition. Here, a group of diapers 110 are placed in a folded condition and held together by holding band 200. An outer encasement 300 may be arranged so as to encase the group of folded diapers 110 held together by band 200. In this manner, the encasement 300 provides protection for both the group of diapers and the band 200. The band may also be arranged outside the encasement 200 as shown in figure 5B. In either case, the band 200 allows to have small thickness of outer encasement 200. In particular, in a case if the absorbent articles 110 are in a compressed or folded condition. Namely, the band 200 is typically capable of absorbing a part of an outward pressure force of the compressed and/or folded articles 110 which would otherwise be needed to be absorbed by the encasement.

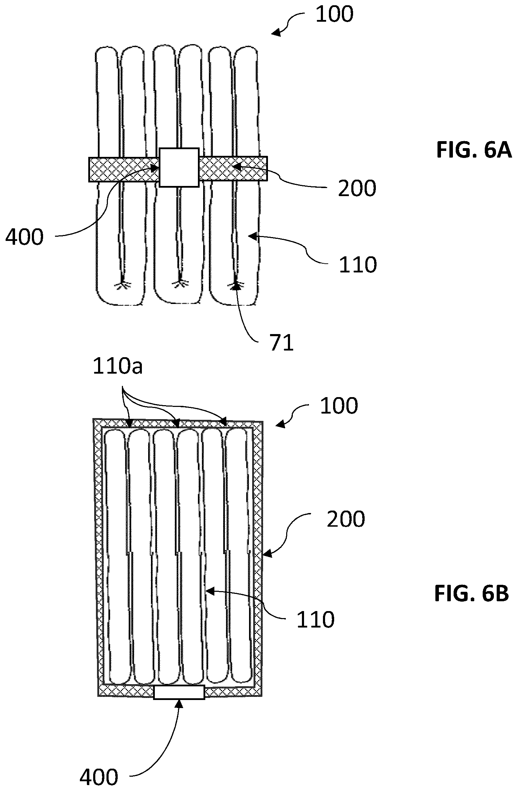

**Figure 6A** shows that band 200 can have a closure mechanism 400. Any suitable closure mechanism can be used. Examples are mechanisms 400 that are or include a pressure sensitive adhesive, a hook and loop system, a snap closure system, a fold-over-flap system or any combination thereof. Figure 6B shows a top view of the pack 100 with the absorbent articles 110 held by band 200. Individual articles 100a can be taken from the pack 100 by opening the band 200 by aid of the closure mechanism 400. Afterwards, the remaining article(s) can be held again by the band 200 by arranging the band 200 again around the remaining article(s) in a closed configuration via the closure mechanism 400.

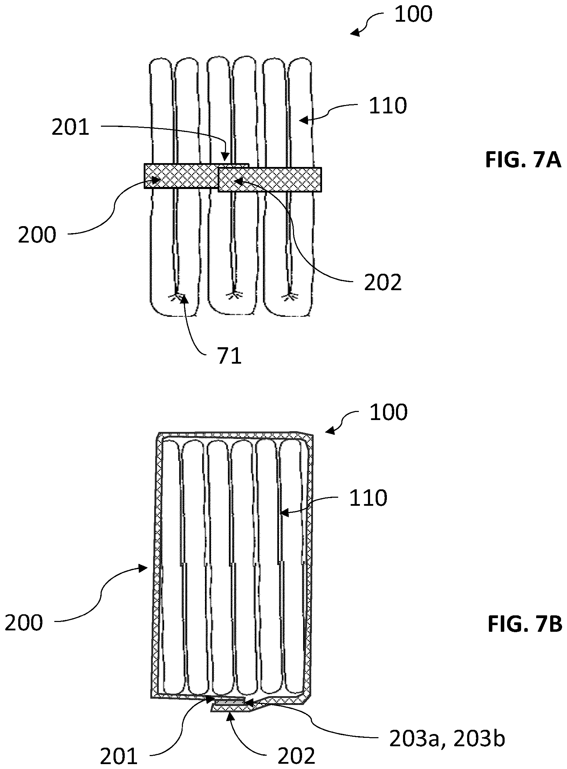

**Figure 7A** shows a pack 100 with a band 200 arranged around a group of articles 110. The band 200 has ends 201, typically opposing ends such as a first end 201 and second end 202, that can be attached to each other by any suitable means. The ends 201, 202 may be configured to be reachable to each other, for example by aid of a pressure sensitive adhesive 203a applied on one or both ends. In a preferred embodiment, the ends 201, 202 may be attached to each other via a seal, such as a heat seal or a ultrasonic seal. In this case, the band 200 may be made of a thermoplastic material and/or the band may be provided with a thermoplastic material arranged to attach the ends 201, 202 under influence of heat or ultrasonic vibrations. The thermoplastic material 203b can for example be applied as a thermoplastic film, coating or strip 203b at one or both ends 201, 202 (see figure 7B). In this manner, the thermoplastic material that can be melted and fused together using highfrequency vibrations (ultrasonic waves) or heat, preferably fused together via ultrasonic waves in view of safety. In particular in case the band 200 is made from paper.

**Figure 7B** shows the pack 100 of figure 7A as seen from a top view. The ends 201 and 202 of the band 200 are sealed together.

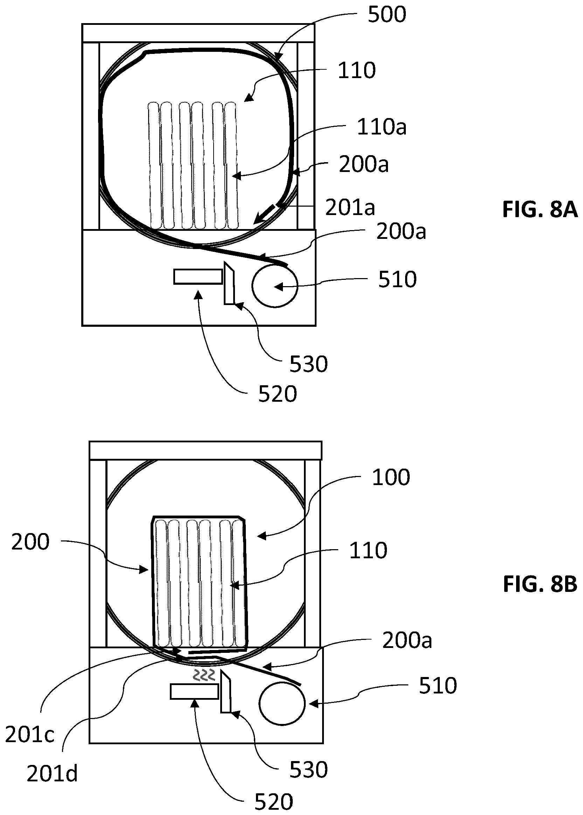

**Figures 8A-8B** illustrate a preferred technique of arranging the band 200 around the one or more absorbent articles 110. The holding band 200 is preferably arranged around the articles by aid of a guiding rail 500 configured for guiding a strip 200a around one or more absorbent articles 110, for example by aid of a conveyor (not shown) conveying the strip 200a around the stack of absorbent articles 110. The strip 200a used to form band 200 may be supplied via supply roll 510. This technique is based on the insight that the band can be arranged in a fast and reliable manner.

**Figure 8A** indicates that first end 201a of the strip 200a can be guided around the stack of articles 110 by aid of the guiding rail 500, the guiding is indicated with the arrow.

**Figure 8B** indicates that after strip 200a is guided around the stack, that the strip 200a can be tightened and sealed by a sealing or a welding process. Preferably via ultrasonic sealing for improved safety, in particular in case the strip 200a is made from paper.

A particular aspect therefore provides in a method for wrapping a strip 201a, around at least one absorbent article 110a to arrange a holding band 200 around said article, wherein the strip is arranged around the at least one article 110a in such a way that at least two strip sections 201c, 201d are brought together and connected to overlap, wherein in an area where the at least two strip sections 201c, 201d are brought together for overlapping, and wherein the two strip sections are sealed together via a welding process, preferably via ultrasonic welding. This aspect is based on the insight that the band can be arranged in a fast and reliable manner. Preferably, the strip 201 is guided and/or positioned via a strip 201a guide device so that the strip is brough around the at least one absorbent article 110a or absorbent articles 110. Preferably, the strip 200a is tensioned before the two strip sections 201c, 201d are welded to each other.

In figure 8B, it is illustrated that the two strip sections 201c, 201d are attached to each other by aid of the ultrasonic waves from ultrasonic transducer 530 which can generate vibrations in the ultrasonic range (typically around 20 kHz or higher). A cutter 530 can be used to cut the strip 200a at a suitable location in order to create from the holding band 200. The strip 200a may be provided with printed information.

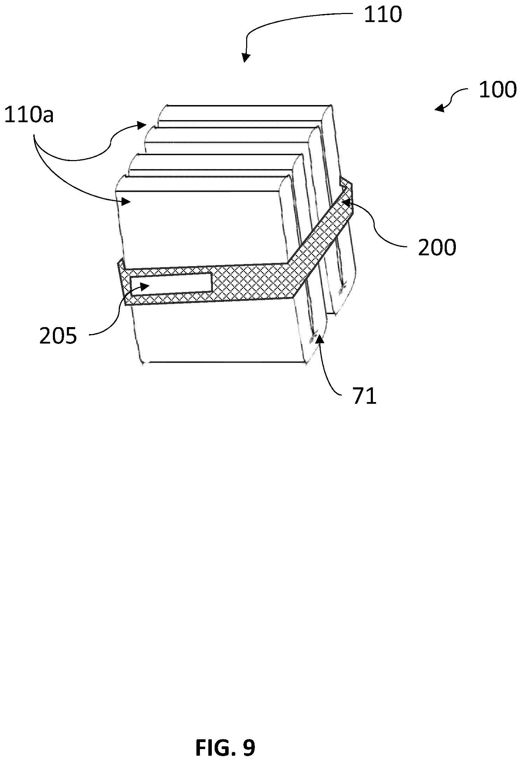

**Figure 9** illustrates a perspective view of pack 100 with a group of absorbent articles 110 that are held together by a holding band 200 which makes direct contact with the absorbent articles 110. Each individual absorbent article 110a in the group of articles 110 are in a folded condition. The diapers 110 are folded via folding line 71.

Figure 9 further indicates that the band 200 has a dedicated surface provided with information related to the absorbent article 110. Preferably, the information is provided on the band 200 before arranging the band 200 around the one or more absorbent articles 110. This way, absorbent articles are better kept away from ink or any other printing factor that may impact the quality of the absorbent article.

Generally, when diapers are held by the holding band 200 in a folded condition it is preferable to fold them so that the sides intended to face the wearer are aligned together. In this manner, comfort of the wearer can be improved as the side touching the wearer was better protected during transport.

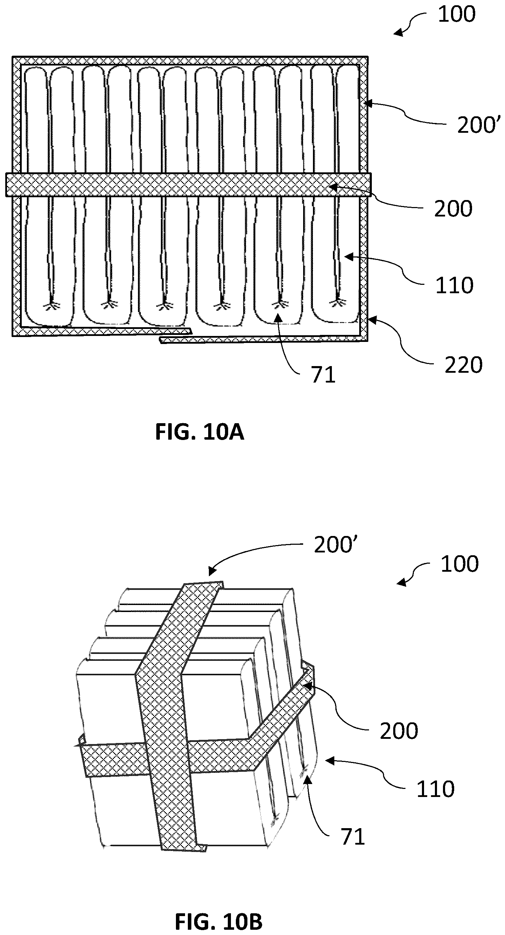

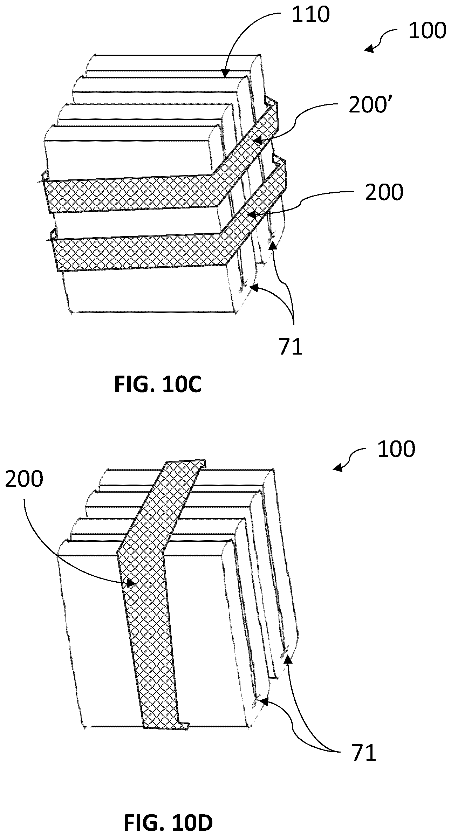

**Figures 10A-10B** illustrate a group of absorbent articles 110 being held by a first holding band 200 and section holding band 200'. The first holding band 200 and section holding band 200' may be arranged around the articles in a crossing (see fig. 10B) or in a mutual parallel manner (see fig. 10C). The first holding band 200 and section holding band 200 can in some embodiments be attached to each other.

In **figure 10A****,** holding band 200' is extends in an imaginary plane which intersects with the folding lines 71. Holding band 200 extends substantially parallel to the folding lines, or with other words, the band 200 extends in a plane that will never intersect with the folding lines or any imaginary extension thereof.

**Figure 10B** shows a perspective view of a pack 100 with absorbent articles 110 held by two holding bands 200, 200' which cross each other.

**Figure 10C** shows a perspective view of a pack 100 with absorbent articles 110 held by two holding bands 200, 200' which are mutually parallel. It has to be understood that the holding band 200 can be arranged in several manners with respect to one or more folding lines of the one or more absorbent article. For example, in figure 10C the absorbent articles 110 are each folded via a folded line 71. Holding bands 200, 200 may be arranged around the articles 110 such that the bands extend substantially parallel to the folding lines. With other words and generally applicable herein, a band 200 extending substantially parallel to the folding line 71 means that the band 200 extends in a pane (not illustrated) that will never cross with the folding line 71 or an imaginary extension thereof (e.g. in figure 10C).

**Figure 10D** illustrates an example wherein the band 200 is extends substantially perpendicular with respect to the holding line, this means that the band 200 extends in a plane that is substantially perpendicular to the folding lines. By having the articles folded and subsequently held by one or more bands 200, 200', several benefits can be achieved, among others, the articles can be transported in a more sustainable way, the one or more bands may also cause the articles to be more appealing to customers and cause the articles to be more recognizable due to a direct view on the articles.

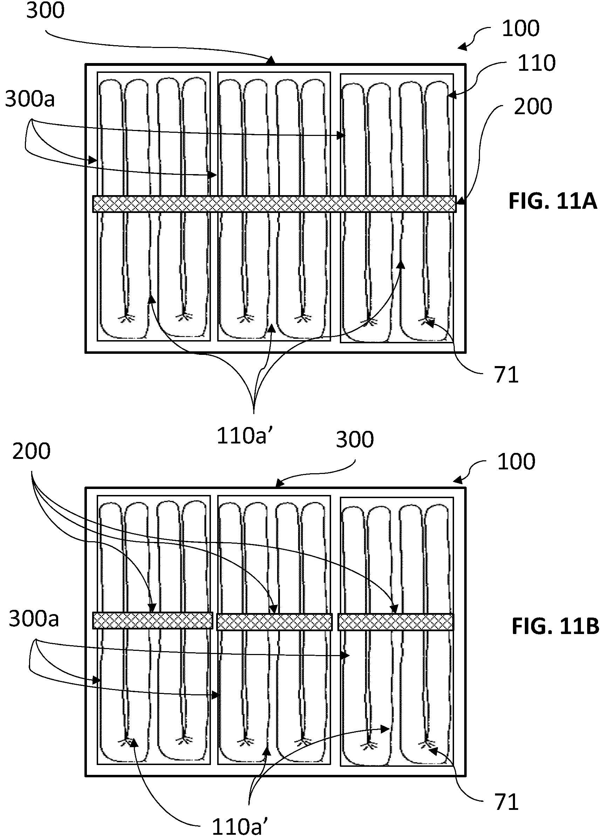

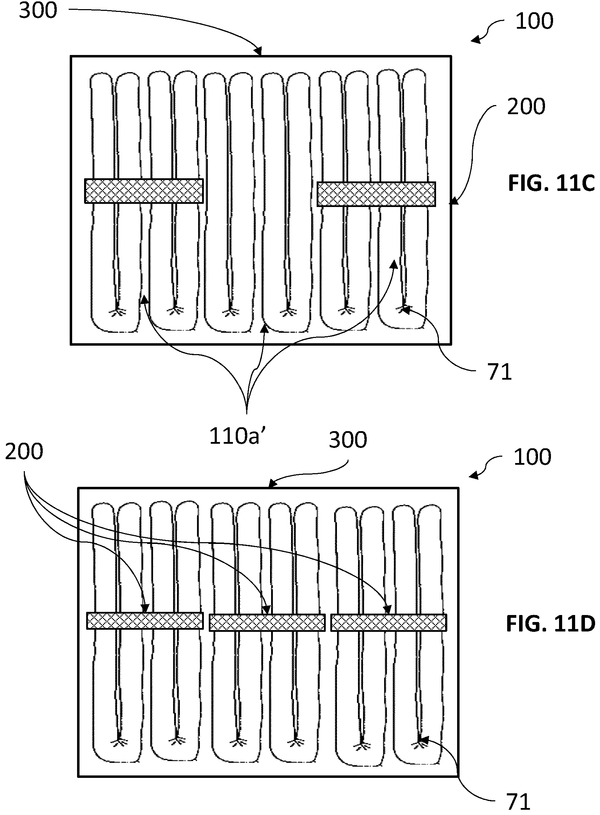

**Figures 11A-11D** illustrate several examples of possible holding band 200 and encasement 300 arrangements. Figure 11A indicates that the absorbent articles 110 can be arranged in sub groups of one or more articles 110a' arranged in sub group. The articles within a sub group 110a' can be encased individually or all together (as shown) by a respective encasement 300a. These encased subgroups of articles 110a' can be held together by one holding band 200 (or by held together individually as shown in figure 11B). Figure 11B indicates that each subgroup 110a' is held together by an individual holding band 200. The articles may be further encased by an additional outer encasement 300 which encases the articles encased by the sub encasements 300a. Notably, the sub encasements 300a are optional and may in some cases not be needed, as indicated with figures 11C and 11D.

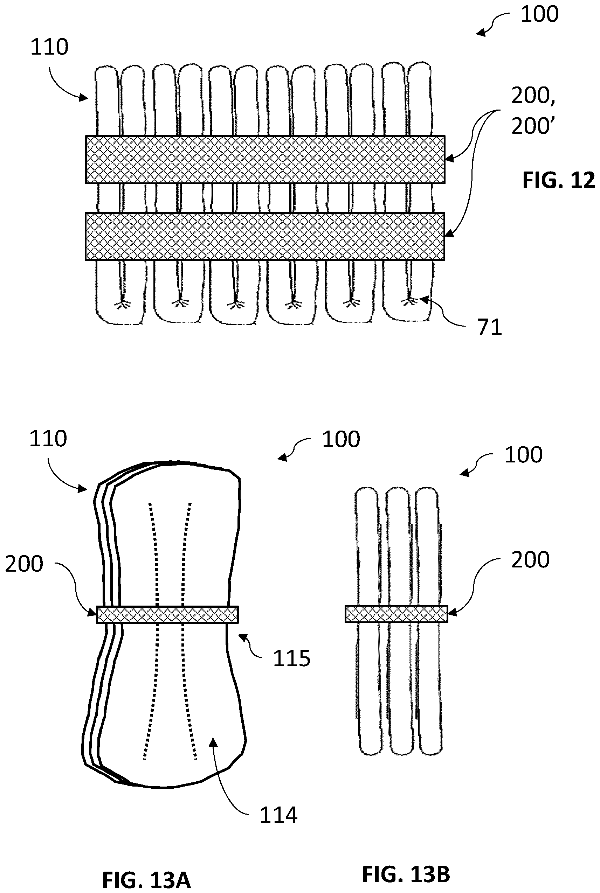

**Figure 12** illustrates a front view of a pack 100 held by two holding bands 200 and 200'. Surprisingly, it was found that two holding bands are highly capable of keeping articles 110 in a preferred condition during transport. A preferred condition may be the folded and/or compressed condition. Indeed, using two holding bands to maintain articles in a preferred condition during transport offers several advantages, including protection from damage, space optimization, ease of handling, reduced wrinkling or tangling, cost savings, and enhanced presentation. One or both of the bands 200 can have any of the features as explained above.

It is preferred that the one or more holding bands 200 are arranged directly adjacent the one or more absorbent article 110, in particular without any encasement in between. In this manner, a highly desired manner of packing the articles is provided with sustainable benefits. Indeed, packaging materials can be reduced and/or can be used more efficiently which benefits sustainability.

**Figures 13A-13B** illustrate a pack of panty liners 110 held by a holding band 200. Indeed, the absorbent articles need not be limited to diapers. Notably, the panty liners may be encased (not shown) whereby a holding band 200 is arranged around the encased panty liners such that the encased panty liners are held together by the holding band.

A panty liner 114 is an absorbent product designed to be worn in the underwear by individuals, usually women, to manage light menstrual flow, vaginal discharge, or for added protection against minor leaks or moisture. The shape of a panty liner can vary slightly between different brands and styles. Preferably, panty liner 114 has a tapered and/or contoured shape to better fit the contours of the body. The shape typically narrows towards a central or middle region 115 of the panty liner. Preferably, the panty liner 114 has a shape having a narrower width in a middle region 115 compared to the end regions of the panty liner 114. The holding band 200 is preferably arranged around the middle region of the panty liner 114. In figure 13A, several panty liners are arranged in a row.

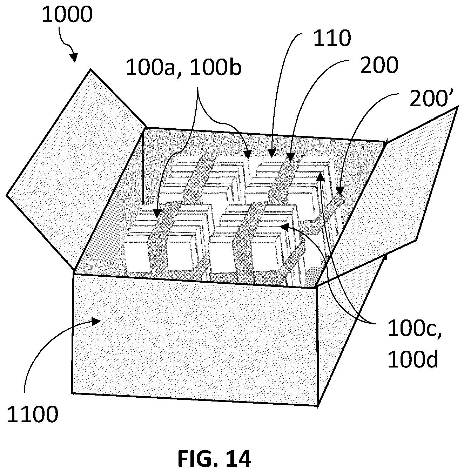

**Figure 14** illustrates a container 1000 containing several absorbent article packs 100a-100c comprising absorbent articles 110. The container 1000 can for example be a box (as shown) or a bag. The container 1000 may contain one or more articles held individually or in group by a holding band 200. The container may also contain one or more groups of articles stacked in one or more stacks, wherein the stacks are each individually held by a holding band 200, or more than one holding band. In figure 14, a plurality of packs 100a - 100d with a group of absorbent articles 110 are each individually held by two holding bands 200, 200'. The bands 200, 200'are preferably arranged directly adjacent the absorbent articles 110 (as shown) without any encasement in between. In this manner, the absorbent articles can be transported in a more sustainable manner as a result of less packaging materials. In other embodiments, an encasement (not shown) is provided for improved hygiene.

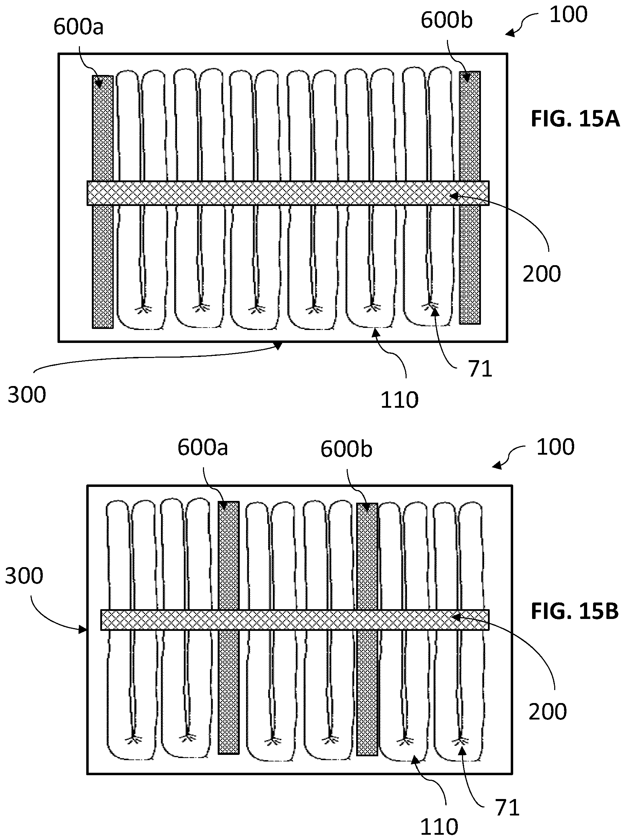

**Figures 15A-15B** each illustrate an absorbent article pack 100 with absorbent articles 110 held by a holding band 200 arranged around the absorbent articles 110. The figures indicate possible arrangements whereby one or more dimensionally stable elements 600a, 600b are included in the pack 100. In figure 15A one or more absorbent articles 110 are placed in between the dimensionally stable elements 600a, 600b placed at the outer positions in the row. Optionally, an outer encasement 300 is provided encasing the absorbent articles and the stable elements both held together via the band 200. Indeed, the stability of pack 100 may be increased by providing one or more dimensionally stable elements 600a, 600b within the encasement 300 and/or within the holding band 200 next to the article(s). For example, one or more dimensionally stable elements may be positioned adjacent and/or in between the absorbent articles. In figure 15B, the dimensionally stable elements 600 are placed in between the articles 110 that are held together via band 200.

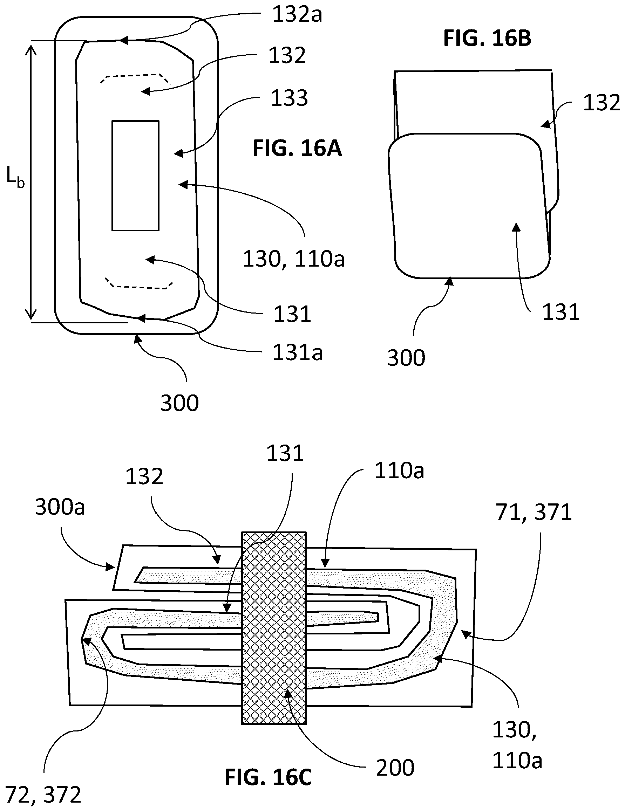

**Figures 16A-16D** shall be used to explain embodiments wherein the absorbent article is folded in a beneficial manner. In particular in case the absorbent article is a panty liner or menstrual pad or any other article designed to absorb bodily fluids such as menstrual blood, vaginal discharge, or urine. Such folded conditions in combination with the holding band allow for a more sustainable way of packing the absorbent articles while at the same time having the article(s) presented in a portable and space efficient manner.

Figure 16A illustrates a panty liner, as absorbent article 110a. The absorbent article has a body 130 with a first end portion 131 and a second end portion 132. Typically, the body 130 extends along a longitudinal axis (not indicated) from a first transversal edge 131a to second transversal edge 132a. The first and second end portions 131, 132 refer to the area's adjacent the transversal edges, typically having a middle portion 133 or crotch region in between. The end portions may each individually extend over 5 to 35 % of the total maximum length L_{b} of the body 130 as measured from the most outward points on the first transversal edge 131a and second transversal edge 132a of the body 130. For example, the first end portion 131 may extend over 25 %, the crotch or middle portion may over 50 % and the second end potion 132 over 25 % of the total maximum length L_{b} the body 130. In figure 16A it is shown that the absorbent article is encased by encasement 300, for example a closed off and sealed bag. Figure 16B shows, as seen from a top view, that the article (not illustrated) within the encasement 300 is folded, more in particular folded such that the first and second end portions overlap.

An example of overlapping end portions is also shown by figure 16C which illustrates a cross section. The folding lines 71 and 371 of the body 130 within this embodiment are perpendicular to the longitudinal axis of the body 130. In this manner, space efficiency, portability can be improved in a sustainable manner. The encasement 300 need not be present, although desired in view of hygiene reasons. In figure 16B and 16C, both the body 130 of the article 110a and the encasement 300 are folded, more in particular such that the folding lines of each correspond with each other. Such folding can be achieved by encasing the article 110a with encasement 300 and subsequently folding both the article 110a and the encasement 300 such that the folding lines 71, 72, 371, 372 of both correspond to each other. The encased article 110a is preferably folded such that the folding lines of the article and encasement extend substantially parallel to the longitudinal axis of the body 130 of the article 110a.

**Figure 16C** further illustrates that the folded encased article 110a are held by holding band 200. In general, the band 200 may be arranged around the folded article 110a in a substantial parallel manner with respect to the folding lines 71, 72 of the body 130 of the folded article 110a (as shown in figure 16C, as well as figure 10C). The band 200 may also be arranged around the folded article 110 in a manner so that the band extends substantially perpendicular to the folding line(s) 71 of the body 130 (e.g. shown in figure 10D). If a folded encasement 300 is present, then the holding band 200 may be arranged parallel to or perpendicular to the folding lines 371, 372 of the folded encasement 300 as well.

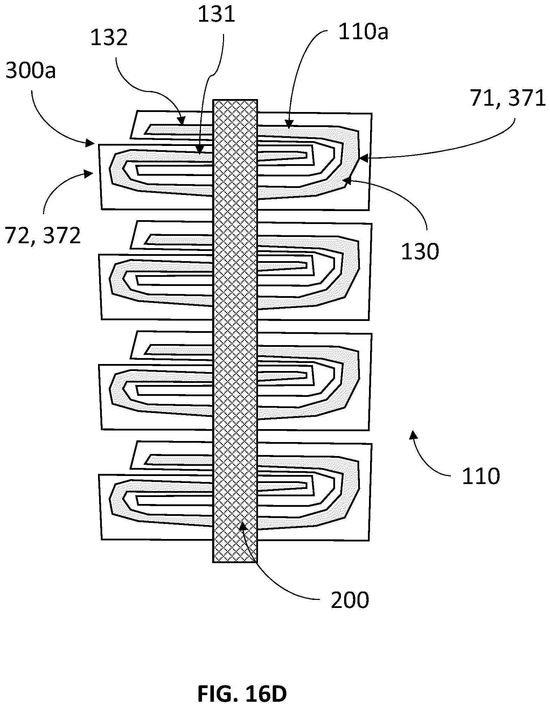

**Figure 16D** further illustrates a plurality of folded absorbent articles 110. The plurality of folded absorbent articles 110 comprise several individual articles 110a that are individually encased, e.g. by encasement 300a as shown. Each article 110a has a body 130 that is folded together with the encasement 300a encasing said article 110a. By folding the body 130 and encasement together, the folding lines 71, 72 of the body 130 correspond to the folding lines 371, 372 of the encasement 300a. In figure 16D, the encased and folded articles are arranged in a stack, the holding band 200 is arranged around said stack.

Hence figure 16D, illustrates a **pack 100** of a plurality absorbent articles 110, wherein each individual article 110a within said plurality of articles 110 is encased by a corresponding individual encasement 300a; and wherein each article 100a and its corresponding individual encasement 300a are folded together thereby forming individually folded encased articles, wherein said individually folded encased articles are held by a holding band 200. Figure 16D further indicates that the folded encased articles 100 are arranged in a stack of encased folded articles and whereby the holding band 200 is arranged around the stack to hold the stack of encased folded articles. By having the encased article(s) in a folded condition, a more portable arrangement can be achieved while the articles can be distributed in a more sustainable manner. For example, the plurality of articles can be distributed with a reduced need for packaging materials (for example a reduced need for other packaging), in particular if the plurality is distributed without other encasements since the holding band is sufficient to transport from factory to consumer while the individual encasements provide for hygiene. The articles 110a and their encasements 300a are preferably folded together such that the folding lines 71, 72, 371, 372 of the article and the respective encasement correspond. Such manner of folding is found to be highly beneficial for absorbent articles being panty liners or menstrual pads. In particular since the overall pack allows for a more sustainable manner of distribution while the individual encasement provides for hygiene. Additionally, by having the articles folded in the manner described, a more portable manner is achieved which is in particular desired when one desires to discreetly carry the absorbent article within a handbag or the like.

The folded condition as explained herein is highly beneficial for panty liners, menstrual pads, which can both be described as sanitary napkins. By folding the articles as described, a more compact outcome may be achieved which makes the article more discreet and more comfortably to carry.

**Figures 17A-17D** show further packs 100 comprising one or more articles 110, in particular wherein the one or more articles are folded via two folding lines 71 and 72.

In this way, a more compact result can be achieved which can be kept secured by aid of the holding band 200. The two folding lines 71 and 72 are preferably parallel to each other. The holding band in figures 17A-17D is shown to be in direct contact with the articles. With other words, no encasement is placed in between the band 200 and the articles 110. In this manner, less packaging material is used which benefits sustainability. In other embodiments, the articles may be encased via an encasement as explained above, for example for improved hygiene.

The above-described embodiments and the figures are purely illustrative and serve only to increase understanding of the invention. The invention will not therefore be limited to the embodiments described herein, but is defined in the claims.

## Claims

1. An absorbent article pack (100) comprising one or more absorbent articles (100a, 100) and at least one holding band (200),
wherein
the at least one holding band (200) is arranged around the one or more absorbent articles (110a, 110) such that the one or more absorbent articles are held together by the at least one holding band.

2. The absorbent article pack (100) according to claim 1, wherein the least one holding band (200) is in direct contact with the one or more absorbent articles (110); and/or wherein the one or more absorbent articles (110) comprise a plurality of absorbent articles which are arranged in a stack, and wherein the at least one holding band is arranged around the stack; and/or wherein one or more absorbent articles (110a, 110) are held together in at least a partially compressed state.

3. The absorbent article pack (100) according to any of the preceding claims, wherein the band (200) has a width (w_{b}) within the range of 0,2 cm to 10 cm, preferably within the range of 0,5 cm and 8 cm, more preferably within 0,5 cm and 6 cm.

4. The absorbent article pack (100) according to any of the preceding claims,
wherein the one or more absorbent articles (110a, 110) are folded and are kept in folded condition by aid of the at least one holding band (200); preferably wherein each of the one or more absorbent articles (110) in folded condition comprise at least two folding lines (71, 72), more preferably wherein said one or more absorbent articles (100) each have a body (130) with a first end portion (131) and a section end portion (132) and wherein for each of the articles, the body (130) is folded such that the first end portion (131) overlaps with the second end portion (132); more preferably wherein the one or more absorbent articles (100),preferably being sanitary napkins, are each individually packed via a folded encasement (300a) folded such that folding lines (371,372) of the encasement (300a) correspond to folding lines (71, 72) of the encased body (130).

5. The absorbent article pack (100) according to any of the preceding claims,
wherein the at least one holding band (200) comprises a dedicated surface (205) provided with information related to the one or more absorbent articles; and/or wherein the absorbent article pack further comprises a first dimensionally stable element (600a), and optionally a second dimensionally stable element (600b), positioned between the at least one holding band (200) and the one or more absorbent articles (100a, 100) and/or between two or more absorbent articles of the one or more absorbent articles (100).

6. The absorbent article pack (100) according to any of the preceding claims,
wherein the at least one holding band (200) comprises a band (200) made of a paper material; preferably wherein said paper material is unbleached and/or uncoated paper and/or has a grammage of between 50 g/m² and 150 g/m², preferably between 60 g/m² and 140 g/m², more preferably between 70 g/m² and 140 g/m², most preferably between 80 g/m² and 120 g/m²; and/or wherein the at least one holding band (200) comprises a band made of plastic material; and/or a band made from a material chosen from the group consisting of polyethylene (PE), polypropylene (PP), polyvinyl chloride (PVC), polyethylene terephthalate (PET), polyamide, ethylene vinyl alcohol (EVOH), polylactic acid (PLA) and/or manufactured form a coextruded film; preferably wherein the at least one holding band is made from polypropylene (PP).

7. The absorbent article pack (100) according to any of the preceding claims,
wherein the at least one holding band (200) is provided with a resealable closure mechanism (400), preferably wherein the resealable closure mechanism (400) comprises any one of a pressure sensitive adhesive, a hook and loop system, a snap closure system, a fold-over-flap system or any combination thereof; and/or wherein the at least one holding band (200) has ends (201, 202) that are sealed together.

8. The absorbent article pack (100) according to any of the preceding claims,
wherein the at least one holding band comprises a first (200) and second holding band (200') which are arranged around the one or more absorbent articles such that the one or more absorbent articles are held together by the first (200) and second holding band (200'), preferably wherein the second holding band (200') crosses over the first holding band (200) and/or wherein the first and second holding band (200, 200') are arranged at a distance from each other, preferably such that the first and second holding band are arranged substantially mutually parallel.

9. The absorbent article pack (100) according to any of the preceding claims,
wherein the pack further comprises an encasement (300) encasing the one or more absorbent articles (110a, 110), preferably wherein the at least one holding band (200) is arranged within the encasement (300); or wherein the at least one holding band (200) is arranged outside the encasement (300); and/or preferably wherein the encasement has a thickness (t) smaller than 500 micron, more preferably wherein the thickness (t) of the encasement (300) is smaller than 50 micron, preferably between 5-50 micron, more preferably 6 - 40 micron, even more preferably between 7-30 micron, most preferably between 8-20 micron, such as 10 micron; and/or preferably wherein the at least one holding band (200) and/or the encasement (300) are made from a recycled material, preferably a post-consumer recycled (PCR) material, such as a recycled PE material or a recycled PET material.

10. The absorbent article pack (100) according to any of the preceding claims, wherein the absorbent article (100a) comprises or the articles within the plurality of absorbent articles (100) each comprise a liquid pervious topsheet, a liquid impervious backsheet, absorbent material positioned between the liquid pervious topsheet and the liquid impervious backsheet, and a bottom layer between the absorbent material and the liquid impervious backsheet, wherein the absorbent material comprises superabsorbent particles and/or cellulosic fluff pulp and wherein the absorbent material is arranged such that one or more channels are formed, wherein less absorbent material per surface area is present in the one or more channels compared to an area around the one or more channels, wherein preferably substantially no absorbent material is present in the one or more channels, wherein the bottom layer is in contact with the absorbent material and has an average density as measured in a decompressed state between 20 and 400 kg/m³, preferably between 20 and 300 kg/m³, more preferably between 20 and 250 kg/m³, and even more preferably between 20 and 200 kg/m³, e.g. between 20 and 120 kg/m³.

11. A container (1000), such as a box (1100) or a bag, containing one or more absorbent article packs (100a, 100b, 100c, 100d) according to any of the preceding claims, wherein a plurality of the absorbent article packs (100a, 100b, 100c, 100d) are arranged directly adjacent to each other within the container and are preferably stacked one above the other and/or are arranged next to each other.

12. A method for packing one absorbent article (110a) or a plurality of absorbent articles (110), the method comprising:
- arranging at least one holding band (200) around the one or more absorbent articles (110a, 110) such that the absorbent article (100a) is, or the more absorbent articles (110) are held together by the at least one holding band, preferably wherein said arranging comprises:
arranging the at least one holding band in an open state around the one or more absorbent articles and sealing together a first and second end of the at least one holding band to achieve a closed state of the at least one holding band, preferably said sealing together is done by heat-sealing or ultrasonic sealing; and/or preferably the method further comprising:
- compressing the one or more absorbent articles (110a, 110),
wherein the one or more absorbent articles (110a, 110) are compressed before being surrounded by the at least one holding band (200); and/or
wherein the one or more absorbent articles (110a, 110) are compressed by being surrounded by the at least one holding band (200) and by subsequently tightening the at least one holding band (200);
wherein preferably the absorbent article (100a) or the plurality of absorbent articles (100) in an uncompressed state take up a total volume V1, wherein the one or more absorbent articles (100a, 100) are compressed such that the total volume V1 of the plurality of absorbent articles is reduced by a factor of 1.05, preferably by a factor of 1.1, more preferably by a factor of 1.2.

13. The method according to any of the preceding method claims, further comprising:
- folding the one or more absorbent articles (110a, 110) before arranging the holding band (200), and subsequently arranging the holding band (200) such that the one or more absorbent articles (110a, 110) are held in a folded condition by the holding band (200); preferably wherein the folding comprises:
- folding the one or more absorbent articles via at least one folding line (71), preferably via at least two folding lines (71, 72); and/or
wherein the one or more absorbent articles are folded and are subsequently encased and/or held by the at least one holding band (200); or wherein the one or more absorbent articles are encased and subsequently folded, preferably folded via at least two folding lines (71, 72) located such that end portions (131, 132) of the encased absorbent article (300a) overlap .

14. The method according to any of the preceding method claims, further comprising:
- encasing the one or more absorbent articles (110a, 110) in an encasement (300), preferably an air-impermeable encasement, preferably wherein said encasement (300) is a shrink film and wherein the method comprises:
- shrinking the encasement (300) after the one or more absorbent articles (110a, 110) are encased within the encasement; and/or
whereby
the holding band (200) is arranged around the encasement (300) after the one or more absorbent articles (110a, 110) are encased therein; or whereby
the holding band (200) is arranged around the one or more absorbent articles (110a before the one or more absorbent articles (110a, 110) are encased such that the holding band (200) and the one or more absorbent articles (110a, 110) are encased within the encasement (300).

15. The method according to any of the previous three method claims, further comprising:
- including a first dimensionally stable element (600a), and optionally a second dimensionally stable element (600b), within the encasement (300).
